# EUROPÄISCHE PATENTANMELDUNG

(11) **EP 0 857 724 A1**
(43) Veröffentlichungstag der Anmeldung: **12.08.1998**
(21) Anmeldenummer: 98101980.5
(22) Anmeldetag: 05.02.1998
(51) Int. Cl.: C07D 335/06, C07D 215/42, C07C 311/07, A61K 31/47

(54) **Sulfonamid-substituierte Verbindungen, Verfahren zu ihrer Herstellung, ihre Verwendung als Medikament oder Diagnostikum**

(30) Priorität: 11.02.1997 DE 19705133
(71) Anmelder: HOECHST AKTIENGESELLSCHAFT, 65929 Frankfurt am Main (DE)
(72) Erfinder: Gerlach, Uwe, Dr., 65795 Hattersheim (DE); Brendel, Joachim Dr., 61118 Bad Vilbel (DE); Lang, Hans Jochen, Dr., 65719 Hofheim (DE); Weidmann, Klaus, Dr., 61476 Kronberg (DE)

(57) **Zusammenfassung**

Verbindungen der Formel I 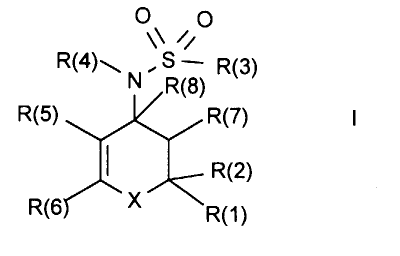 worin R(1) bis R(8) und X die in den Ansprüchen angegebenen Bedeutungen haben, sind wertvoll zur Herstellung von Medikamenten mit K⁺-Kanal blockierenden Wirkung; insbesondere zur Herstellung eines Medikaments zur Behandlung oder Prophylaxe der stimulierten Magensäuresekretion; von Ulcera des Magens und des intestinalen Bereiches; der Refluxoesophagitis; der Diarrhoe; aller Typen von Arrhythmien einschließlich ventrikulärer und supraventrikulärer Arrhythmien; und von Reentry-Arrhythmien und zur Prophylaxe des plötzlichen Herztodes infolge von Kammerflimmern.

## Beschreibung

Die Erfindung betrifft Verbindungen der Formel I 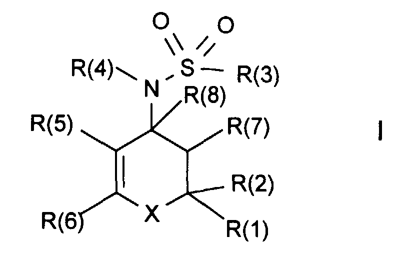 worin bedeuten:
- X: -[S(O) _{Null, 1 oder 2}]-, -NR(9)-, -[CR(9)R(23)]- oder CO-;
- R(9): Wasserstoff oder -(CₙH₂ₙ)-R(10);
- n: Null, 1, 2, 3, 4, 5, 6, 7 oder 8;
- R(10): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH = CH-, -C≡C-, -CO-, -CO-O-, -[SO_{Null, 1 oder 2}]- oder -NR(11)-;
- R(11): Wasserstoff, Methyl oder Ethyl;
oder
- R(10): Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
oder
- R(9): gemeinsam mit R(1) eine Bindung;
- R(23): Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
- R(24): Wasserstoff, Methyl oder Ethyl;
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 2, 3, 4, 5, 6, 7, 8, 9 oder 10 C-Atomen;
- R(3): R(12)-CₐH₂ₐ[NR(13)]ₘ-;
- R(12): Wasserstoff oder Cycloalkyl mit 3, 4, 5, 6, 7 oder 8 C-Atomen, CF₃, C₂F₅ oder C₃F₇;
- a: Null, 1, 2, 3, 4, 5, 6, 7, 8, 9 oder 10;
- m: Null oder 1;
- R(13): Wasserstoff oder Alkyl mit 1, 2, 3, 4, 5 oder 6 C-Atomen;
oder
- R(12) und R(13): gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
- R(11): Wasserstoff, Methyl oder Ethyl;
- R(4): R(14)-CᵣH₂ᵣ;
- r: 1, 2, 3, 4, 5, 6, 7, 8, 9, 10, 11, 12, 13, 14, 15, 16, 17, 18, 19 oder 20;
- R(14): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
- R(3) und R(4): gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
- R(5) und R(6): gemeinsam -CR(15)=CR(16)-CR(17)=CR(18)-, -CR(15)=CR(16)-CR(17)=N-, -CR(15)=CR(16)-N=CR(18)-, -CR(15)=N-CR(17)=N-, -CR(15)=N-N=CR(18)-, -N=CR(16)-CR(17)=N- oder -S-CR(15)=CR(16)-;
- R(15), R(16), R(17) und R(18): unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(19) und R(20): unabhängig voneinander Wasserstoff oder Alkyl mit 1, 2 oder 3 C-Atomen;
- R(21): Wasserstoff, Methyl, Ethyl, Phenyl oder
-CᵤH₂ᵤ-NR(19)R(20);
- u: 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- R(22): Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- s: Null, 1, 2, 3, 4, 5 oder 6;
- Z: -[S(O)_{Null, 1 oder 2}]-, -CO-, -SO₂-NR(11)-, -SO₂-O-, -O-, -NR(11)- oder -[CO-NR(11)]-;
- R(7): Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
- R(8): Wasserstoff oder Alkyl mit 1, 2, 3, 4, 5 oder 6 C-Atomen;
und deren physiologisch verträgliche Salze.

Bevorzugt sind Verbindungen der Formel I, in welcher bedeuten:
- X: -S(O)_{Null, 1 oder 2}]-, -NR(9)- oder -[CR(9)R(23)]- ;
- R(9): Wasserstoff oder (CₙH₂ₙ)-R(10);
- n: Null, 1, 2, 3, 4, 5, 6, 7 oder 8;
- R(10): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
- R(11): Wasserstoff, Methyl oder Ethyl;
oder
- R(10): Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und
Methylsulfonylamino;
oder
- R(9): gemeinsam mit R(1) eine Bindung;
- R(23): Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
- R(24): Wasserstoff, Methyl oder Ethyl;
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 2, 3, 4, 5, 6, 7, 8, 9 oder 10 C-Atomen;
- R(3): R(12)-CₐH₂ₐ[NR(13)]ₘ-;
- R(12): Wasserstoff oder Cycloalkyl mit 3, 4, 5, 6, 7 oder 8 C-Atomen, CF₃, C₂F₅ oder C₃F₇;
- a: Null, 1, 2, 3, 4, 5, 6, 7, 8, 9 oder 10;
- m: Null oder 1;
- R(13): Wasserstoff oder Alkyl mit 1, 2, 3, 4, 5 oder 6 C-Atomen;
oder
- R(12) und R(13): gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann; R(11) Wasserstoff, Methyl oder Ethyl;
- R(4): R(14)-CᵣH₂ᵣ;
- r: 1, 2, 3, 4, 5, 6, 7, 8, 9, 10, 11, 12, 13, 14, 15, 16, 17, 18, 19 oder 20;
- R(14): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
- R(3) und R(4): gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
- R(5) und R(6): gemeinsam -CR(15)=CR(16)-CR(17)=CR(18)- oder
-S-CR(15)=CR(16)-;
- R(15), R(16), R(17) und R(18): unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(19) und R(20): unabhängig voneinander Wasserstoff oder Alkyl mit 1, 2 oder 3 C-Atomen;
- R(21): Wasserstoff, Methyl, Ethyl, Phenyl oder -CᵤH₂ᵤ-NR(19)R(20);
- u: 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- R(22): Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- s: Null, 1, 2, 3, 4, 5 oder 6;
- Z: -[S(O)_{Null, 1 oder 2}]-, -CO-, -SO₂-NR(11)-, -SO₂-O-, -O-, -NR(11)- oder -[CO-NR(11)]-;
- R(7): Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
- R(8): Wasserstoff oder Alkyl mit 1, 2, 3, 4, 5 oder 6 C-Atomen.
und deren physiologisch verträgliche Salze.

Besonders bevorzugt sind Verbindungen der Formel 1, in der bedeuten:
- X: -NR(9)- oder -[CR(9)R(23)]-;
- R(9): Wasserstoff oder -(CₙH₂ₙ)-R(10);
- n: Null, 1, 2, 3, 4, 5, 6, 7 oder 8;
- R(10): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
- R(11): Wasserstoff, Methyl oder Ethyl;
oder
- R(10): Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und
Methylsulfonylamino;
oder
- R(9): gemeinsam mit R(1) eine Bindung;
- R(23): Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
- R(24): Wasserstoff, Methyl oder Ethyl;
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 2, 3, 4, 5, 6, 7, 8, 9 oder 10 C-Atomen;
- R(3): R(12)-CₐH₂ₐ[NR(13)]ₘ-;
- R(12): Wasserstoff oder Cycloalkyl mit 3, 4, 5, 6, 7 oder 8 C-Atomen, CF₃, C₂F₅ oder C₃F₇;
- a: Null, 1, 2, 3, 4, 5, 6, 7, 8, 9 oder 10;
- m: Null oder 1;
- R(13): Wasserstoff oder Alkyl mit 1, 2, 3, 4, 5 oder 6 C-Atomen;
oder
- R(12) und R(13): gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann; R(11) Wasserstoff, Methyl oder Ethyl;
- R(4): R(14)-CᵣH₂ᵣ;
- r: 1, 2, 3, 4, 5, 6, 7, 8, 9 oder 10;
- R(14): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
- R(3) und R(4): gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
- R(5) und R(6): gemeinsam -CR( 15)=CR(16)-CR(17)=CR(18)-;
- R(15), R(16), R(17) und R(18): unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(19) und R(20): unabhängig voneinander Wasserstoff oder Alkyl mit 1, 2 oder 3 C-Atomen;
- R(21): Wasserstoff, Methyl, Ethyl, Phenyl oder -CᵤH₂ᵤ-NR(19)R(20);
- u: 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- R(22): Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- s: Null, 1, 2, 3, 4, 5 oder 6;
- Z: -[S(O)_{Null, 1 oder 2}]-, -CO-, -SO₂-NR(11)-, -SO₂-O-, -O-, -NR(11]- oder -[CO-NR(11)]-;
- R(7): Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
- R(8): Wasserstoff oder Alkyl mit 1, 2, 3, 4, 5 oder 6 C-Atomen;
und deren physiologisch verträgliche Salze.

Ganz besonders bevorzugt sind Verbindungen der Formel I, in der bedeuten:
- X: -NR(9)- oder -[CR(9)R(23)]-;
- R(9): Wasserstoff oder -(CₙH₂ₙ)-R(10);
- n: Null, 1, 2, 3, 4, 5, 6, 7 oder 8;
- R(10): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
- R(11): Wasserstoff, Methyl oder Ethyl;
oder
- R(10): Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
- R(23): Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
- R(1) und R(2): gemeinsam eine Alkylenkette mit 2, 3, 4, 5, 6, 7, 8, 9 oder 10 C-Atomen;
- R(3): R(12)-CₐH₂ₐ[NR(13)]ₘ-;
- R(12): Wasserstoff oder Cycloalkyl mit 3, 4, 5, 6, 7 oder 8 C-Atomen, CF₃, C₂F₅ oder C₃F₇;
- a: Null, 1, 2, 3, 4, 5, 6, 7, 8, 9 oder 10;
- m: Null oder 1;
- R(13): Wasserstoff oder Alkyl mit 1, 2, 3, 4, 5 oder 6 C-Atomen;
oder
- R(12) und R(13): gemeinsam eine Alkylengruppe mit 4, 5, 6, 7 oder 8 C-Atomen,
wobei eine CH₂-Gruppe der Alkylengruppe durch -O-, -[SO_{Null, 1 oder 2}]-, -CO- oder -NR(11)- ersetzt sein kann; R(11) Wasserstoff, Methyl oder Ethyl;
- R(4): R(14)-CᵣH₂ᵣ;
- r: 1, 2, 3, 4, 5, 6, 7, 8, 9, 10, 11, 12, 13, 14, 15, 16, 17, 18, 19 oder 20;
- R(14): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
- R(3) und R(4): gemeinsam eine Alkylenkette mit 3, 4, 5, 6, 7 oder 8 C-Atomen, wobei eine CH₂-Gruppe der Alkylenkette durch -O-,
-[SO_{Null, 1 oder 2}]-, -CO- oder -NR(11)- ersetzt sein kann;
- R(5) und R(6): gemeinsam -CR(15)=CR(16)-CR(17)=CR(18)-;
- R(15), R(16), R(17) und R(18): unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(19) und R(20): unabhängig voneinander Wasserstoff oder Alkyl mit 1, 2 oder 3 C-Atomen;
- R(21): Wasserstoff, Methyl, Ethyl, Phenyl oder
-CᵤH₂ᵤ-NR(19)R(20);
- u: 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- R(22): Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- s: Null, 1, 2, 3, 4, 5 oder 6;
- Z: -[S(O)_{Null, 1 oder 2}]-, -CO-, -SO₂-NR(11)-, -SO₂-O-, -O-, -NR(11)- oder -[CO-NR(11)]-;
- R(7): Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
- R(8): Wasserstoff oder Alkyl mit 1, 2, 3, 4, 5 oder 6 C-Atomen.
und deren physiologisch verträgliche Salze.

Ganz speziell bevorzugt sind Verbindungen der Formel I, in der bedeuten:
- X: -NR(9)- oder -[CR(9)R(23)]-;
- R(9): Wasserstoff oder -(CₙH₂ₙ)-R(10);
- n: Null, 1, 2, 3 oder 4;
- R(10): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
- R(11): Wasserstoff, Methyl oder Ethyl;
oder
- R(10): Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und
Methylsulfonylamino;
- R(23): Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
- R(1) und R(2): unabhängig voneinander Wasserstoff, CF₃, C₂F₅, C₃F₇, Alkyl mit 1 oder 2 C-Atomen;
oder
- R(1) und R(2): gemeinsam eine Alkylenkette mit 2, 3, 4, 5 oder 6 C-Atomen;
- R(3): R(12)-CₐH₂ₐ[NR(13)]ₘ-;
- R(12): Wasserstoff oder Cycloalkyl mit 3, 4, 5 oder 6 C-Atomen, CF₃, C₂F₅ oder C₃F₇;
- a: Null, 1, 2, 3, 4, 5 oder 6;
- m: Null;
- R(13): Wasserstoff oder Alkyl mit 1, 2, 3, 4, 5 oder 6 C-Atomen;
oder
- R(12) und R(13): gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann; R(11) Wasserstoff, Methyl oder Ethyl;
- R(4): R(14)-CᵣH₂ᵣ;
- r: 1, 2, 3, 4, 5, 6, 7, 8, 9 oder 10;
- R(14): Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
- R(3) und R(4): gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
- R(5) und R(6): gemeinsam -CR(15)=CR(16)-CR(17)=CR(18)-;
- R(15) und R(18): Wasserstoff;
- R(16) und R(17): unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
- R(19) und R(20): unabhängig voneinander Wasserstoff oder Alkyl mit 1, 2 oder 3 C-Atomen;
- R(21): Wasserstoff, Methyl, Ethyl, Phenyl oder -CᵤH₂ᵤ-NR(19)R(20);
- u: 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- R(22): Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
- s: Null, 1, 2, 3, 4, 5 oder 6;
- Z: -[S(O)_{Null, 1 oder 2}]-, -CO-, -SO₂-NR(11)-, -SO₂-O-, -O-, -NR(11)- oder -[CO-NR(11)]-;
- R(7): Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
- R(8): Wasserstoff oder Alkyl mit 1, 2, 3, 4, 5 oder 6 C-Atomen;
sowie ihre pharmazeutisch verträglichen Salze.

Enthalten die Verbindungen I eine saure oder basische Gruppe bzw. einen basischen Heterocyclus, so sind auch die entsprechenden, pharmakologisch und toxikologisch verträglichen Salze Gegenstand der Erfindung. So können die Verbindungen I, die eine oder mehrere -COOH-Gruppen tragen, beispielsweise als Alkalisalze, vorzugsweise als Natrium- oder Kaliumsalze verwendet werden. Verbindungen I, die eine basische, protonierbare Gruppe oder einen basischen heterocyclischen Rest tragen, können auch in Form ihrer organischen oder anorganischen, pharmakologisch und toxikologisch verträglichen Säureadditionssalze verwendet werden, beispielweise als Hydrochloride, Methansulfonate, Acetate, Lactate, Maleinate, Fumarate, Malate, Gluconate usw. Enthalten die Verbindungen I eine saure und basische Gruppe im gleichen Molekül, so gehören neben den geschilderten Salzformen auch innere Salze, sogenannte Betaine, zur der Erfindung.

Enthalten die Substituenten der Verbindungen I Gruppen mit unterschiedlichen stereochemischen Möglichkeiten, so gehören auch die einzelnen möglichen Stereoisomeren zur Erfindung, so daß im Falle von optischer Isomerie die einzelnen reinen Enantiomeren sowie beliebige Stoffgemische dieser optischen Isomeren Teil der Erfindung sind.

Alkyl- und Alkylenreste können geradkettig oder verzweigt sein.

Die Verbindungen der Formel I sind durch unterschiedliche chemische Verfahren herstellbar, die ebenfalls Teil der Erfindung sind.

So erhält man eine Verbindung der Formel I, indem man
a) eine Verbindung der Formel II 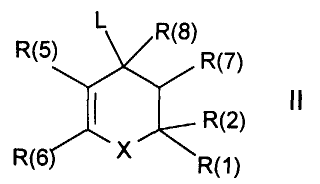 worin R(1), R(2), R(5), R(6), R(7), R(8) und X die angegebene Bedeutung besitzen und L eine übliche nucleofuge Fluchtgruppe, insbesondere F, Cl, Br, I, MeSO₂-O-, einen p-Toluolsulfonyloxy-Rest, oder R(7) und L gemeinsam ein Epoxidring sind, bedeutet,
   mit einem Sulfonamid oder dessen Salz der Formel III 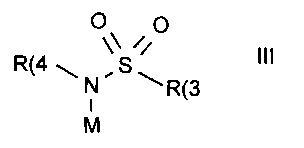 in an sich bekannter Weise umsetzt, worin R(3) und R(4) die angegebene Bedeutung besitzen und M für Wasserstoff oder vorzugsweise für ein Metallatom, besonders bevorzugt für Lithium, Natrium oder Kalium steht;
   oder daß man
b) eine Verbindung der Formel IV 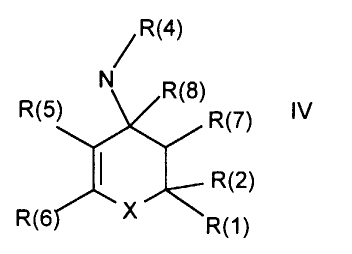 worin R(1), R(2), R(4), R(5), R(6), R(7), R(8) und X die angegebene Bedeutung besitzen,
   mit einem Sulfonsäure-Derivat der Formel V 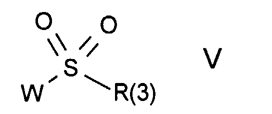 umsetzt, worin R(3) die angegebene Bedeutung besitzt und W eine nucleofuge Fluchtgruppe, wie Fluor, Brom, 1-Imidazolyl, insbesondere aber Chlor bedeutet;
   oder daß man
c) eine Verbindung der Formel VI 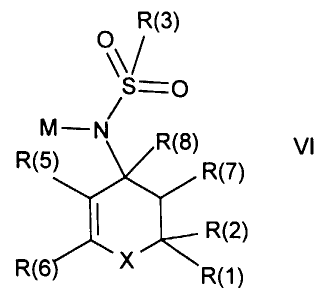 worin R(1), R(2), R(3), R(5), R(6), R(7), R(8), X und M die angegebene Bedeutung besitzen, in an sich bekannter Weise mit einem Alkylierungsmittel der Formel VII

   R(4)-L VII

   im Sinne einer Alkylierungsreaktion umsetzt, worin R(4) mit Ausnahme von Wasserstoff sowie L die angegebene Bedeutung besitzen;
   oder daß man
d) in einer Verbindung der Formel I 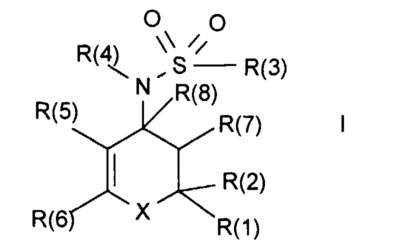 worin R(1) bis R(4), R(7), R(8) und X die angegebene Bedeutung besitzen, in mindestens einer der Positionen R(15), R(16), R(17), R(18) des Ringsystems R(5)-R(6) eine elektrophile Substitutionsreaktion durchführt, sofern diese Position Wasserstoff bedeutet und die restlichen Substituenten R(5) bis R(8) die angegebene Bedeutung besitzen.

### Verfahrensweise a)

beschreibt die Reaktion eines Sulfonamids bzw. eines seiner Salze der Formel III mit einem reaktiven Heterocyclus der Formel II. Da die Reaktion eines Sulfonamides III aus der Salzform heraus erfolgt, muß bei Verwendung eines freien Sulfonamids (Formel III, M = H) durch Einwirkung einer Base ein Sulfonamidsalz (Formel III, M = Kation) erzeugt werden, das sich durch höhere Nucleophilie und damit durch höhere Reaktivität auszeichnet. Setzt man freies Sulfonamid (M = H) ein, so erfolgt die Deprotonierung des Sulfonamids zum Salz in situ unter bevorzugter Verwendung solcher Basen, die selbst nicht oder nur wenig alkyliert werden, wie Natriumcarbonat, Kaliumcarbonat, ein sterisch stark gehindertes Amin, z. B. Dicyclohexylamin, N,N,N-Dicyclohexyl-ethylamin oder andere starke Stickstoffbasen mit geringer Nucleophilie, beispielsweise DBU, N,N',N'''-Triisopropylguanidin etc. Es können allerdings auch andere üblich verwendete Basen für die Reaktion eingesetzt werden, wie Kalium-tert.butylat, Natriummethylat, Alkalihydrogencarbonate, Alkalihydroxide, wie beispielweise LiOH, NaOH oder KOH, oder Alkalihydroxide, beispielsweise Ca(OH)₂.

Dabei arbeitet man vorzugsweise in polaren organischen Lösungsmitteln wie Dimethylformamid, Dimethylacetamid, Tetramethylharnstoff, Hexamethylphosphorsäuretriamid, Tetrahydrofuran, Dimethoxyethan, Toluol, einem halogenierten Kohlenwasserstoff wie Chloroform oder Methylenchlorid usw. Prinzipiell kann aber auch in polaren protischen Lösungsmitteln gearbeitet werden, wie Wasser, Methanol, Ethanol, Isopropanol, Ethylenglykol oder dessen Oligomeren und deren entsprechenden Halbethern und Ethern. Die Reaktion wird in einem bevorzugten Temperaturbereich von -10 bis 140°C, besonders bevorzugt von 20 bis 100°C durchgeführt. In günstiger Weise kann Verfahrensweise a) auch unter den Bedingungen einer Zweiphasenkatalyse durchgeführt werden.

Die Verbindungen der Formel II gewinnt man nach literaturbekannten Methoden, beispielsweise aus den entsprechenden ungesättigten Verbindung X 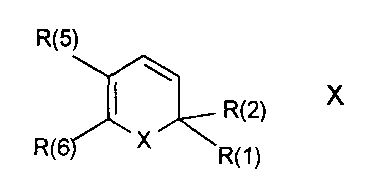 durch Einwirkung eines anorganischen oder organischen Peroxides, wie beispielsweise H₂O₂, MCPBA, Peressigsäure. Die Anlagerung von Halogen ist auch durch die Reaktion von X mit NCS, NBS, Chlor oder Brom in wäßrigen Lösungsmitteln möglich. Vorteilhaft arbeitet man in einem Lösungsmittel, das ausreichend inert gegenüber diesen halogenierenden oder oxidierenden Reagenzien ist, wie beispielsweise in DMSO oder halogenierten Kohlenwasserstoffen wie z.B. Chloroform, Methylenchlorid.

### Verfahrensweise b)

beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechenden Sulfonamid-Derivat der Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.
Die Amino-Derivate der Formel IV erhält man in an sich literaturbekannter Weise bevorzugt durch Reaktion der reaktiven Verbindungen der Formel II mit R(1), R(2), R(5), R(6) und L in der angegebenen Bedeutung, entweder mit Ammoniak oder einem Amin der Formel XI

R(4)-NH₂ XI

mit R(4) in der angegebenen Bedeutung.

### Verfahrensweise c)

repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen.
Die Herstellung der Sulfonamid-Derivate VI und deren Vorprodukte wurden bereits bei Verfahrensweise b) beschrieben. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

### Verfahrensweise d)

beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten.
Bevorzugte Substitutionsreaktionen sind
1. die aromatische Nitrierung zur Einführung einer oder mehrerer Nitrogruppen, sowie deren nachfolgende Reduktion zu NH₂-,
2. die aromatische Halogenierung insbesondere zur Einführung von Chlor, Brom oder Jod,
3. die Chlorsulfonierung zur Einführung einer Chlorsulfonylgruppe durch Einwirkung von Chlorsulfonsäure,
4. die Friedel-Crafts Acylierungsreaktion zur Einführung eines Acylrestes R(16)-CₛH₂ₛ-CO- oder eines Sulfonylrestes R(16)-CₛH₂ₛ-SO₂- durch Einwirkung der entsprechenden Säurechloride R(16)-CₛH₂ₛ-CO-Cl bzw. R(16)-CₛH₂ₛ-SO₂-Cl in Gegenwart einer Lewis-Säure als Friedel-Crafts-Katalysator, vorzugsweise von wasserfreiem Aluminiumchlorid.

Die Verbindungen I sind verwandt mit der in der letzten Dekade in der Arzneimittelchemie intensiv bearbeiteten Klasse der 4-Acylaminochroman-Derivate, insbesondere von 2,2-Dialkyl-4-acylamino-3-chromanolen. Prominentester Vertreter derartiger 4-Acylaminochromane ist das Cromakalim der Formel XII 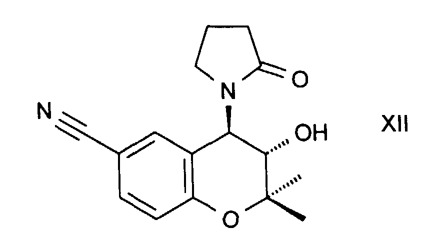 und zahlreiche, sich von diesem Präparat ableitenden Folgepräparate (z.B. Edwards and Weston, TIPS 11, 417 - 422 (1990), "Structure Activity Relationships of K⁺channel openers"

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle ("Hyperpolarisation") wirkt über Sekundärmechanismen der Erhöhung von intrazellulärem Ca²⁺ und damit einer Zellaktivierung, z. B. einer Muskelkontraktion, entgegen.

Im Gegensatz zu diesen 4-Acylaminochroman-Derivaten, die wie erwähnt als Öffner des ATP-sensitiven K⁺ Kanals identifiziert wurden, zeigen die erfindungsgemäßen Verbindungen der Formel I mit der 4-Sulfonylaminostruktur überraschenderweise eine starke und spezifische blockierende (schließende) Wirkung auf einen K⁺ Kanal, der durch cyclisches Adenosinmonophosphat (cAMP) geöffnet wird und sich grundlegend vom erwähnten K⁺(ATP)-Kanal unterscheidet. Neuere Untersuchungen zeigen vielmehr, daß dieser am Dickdarm identifizierte K⁺(cAMP) -Kanal mit hoher Wahrscheinlichkeit identisch mit dem am Herzmuskel identifizierten I_{Ks} -Kanal ist. Infolge dieser Blockierung des K⁺(cAMP) -Kanals (= I_{Ks}-Kanals) entwickeln die Verbindungen im lebenden Organismus pharmakologische Wirkungen von hoher therapeutischer Verwertbarkeit.

So zeichnen sich die Verbindungen als neue Wirkstoffklasse potenter Inhibitoren der stimulierten Magensäuresekretion aus. Die Verbindungen der Formel I sind somit wertvolle Medikamente zur Behandlung von Ulcera des Magens und des intestinalen Bereiches, beispielsweise des Duodenums. Sie eignen sich ebenfalls infolge ihrer starken Magensaftsekretions-hemmenden Wirkung als ausgezeichnete Therapeutika zur Behandlung der Refluxoesophagitis.

Die Verbindungen zeichnen sich weiterhin durch eine antidiarrhoische Wirkung aus und sind deshalb als Arzneimittel zur Behandlung von Durchfall-Erkrankungen geeignet.

Weiterhin können die Verbindungen I als Arzneimittel zur Behandlung und Verhinderung aller Typen von Arrhythmien einschließlich ventrikulärer und supraventrikulärer Arrhythmien verwendet werden. Sie können insbesondere angewandt werden zur Kontrolle von Reentry-Arrhythmien, Vorhof-Flimmern und zur Verhinderung des plötzlichen Herztodes infolge von Kammerflimmern.

Inzwischen existieren Publikationen, aus denen eine Korrelation zwischen I_{sK}-Kanal-inhibitorischer Wirkung und dem Unterbinden von lebensbedrohlichen cardialen Arrhythmien beschrieben wird, wie sie beispielsweise durch β-adrenerge Hyperstimulation ausgelöst werden (z. B. T. J. Colatsky, C. H. Follmer und C .F. Starmer: *"* Channel Specificity in Antiarrhythmic Drug Action; Mechanism of potassium channel block and its role in suppressing and aggravating cardiac arrhythmias*",* Circulation (1990) 82: 2235 - 2242; A. E. Busch, K. Malloy, W. J. Groh, M. D. Varnum, J. P. Adelman und J. Maylie; *"*The novel class III antiarrhythmics NE-10064 and NE-10133 inhibit I_{sK} channels in Xenopus oocytes and I_{K}S in guinea pig cardiac myocytes*",* Biochem. Biophys. Res. Commun. (1994) 202: 265 - 270).

2-Carboxy-4-amido-tetrahydroquinolone sind der Gegenstand einer Publikation (P. D. Leeson et al, J. Med. Chem. 35 (1992) 1954 - 1568) und der Europäischen Offenlegungsschrift 386 839. Die beschriebenen Verbindungen sind sowohl strukturell andersartig als auch in ihrer pharmakologischen Eigenschaften nicht vergleichbar und besitzen somit einen anderen therapeutischen Anwendungsbereich.

Arzneimittel, die eine erfindungsgemäße Verbindung I enthalten, können oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes des Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen; aber auch von Art und Stärke der zu behandelnden Krankheit sowie von Geschlecht, Alter, Gewicht und individueller Ansprechbarkeit des zu behandelnden Säugers ab.

Im Durchschnitt beträgt die tägliche Dosis einer Verbindung der Formel I bei einem etwa 75 kg schweren Patienten mindestens 0,001 mg, vorzugsweise 0,1 mg, insbesondere mindestens 10 mg bis höchstens 100 mg, vorzugsweise höchstens 1 g pro kg.

Erklärung der im Text verwendeten Abkürzungen
- DMA: Dimethylacetamid
- HMPT: Hexamethylphosphorsäuretriamid
- TMU: Tetramethylharnstoff
- hr: Stunde(n)
- M: Mol
- MCPBA: m-Chlorperbenzoesäure
- mM: Millimol
- min: Minuten
- TEA: Triethylamin
- THF: Tetrahydrofuran
- NBS: N-Brom-Succinimid
- NCS: N-Chlor-Succinimid
- EE: Ethylacetat

### Beispiel 1: N-Methyl-N-(7-nitro-1,2,3,4-tetrahydro-naphthalin-1-yl)-methansulfonamid

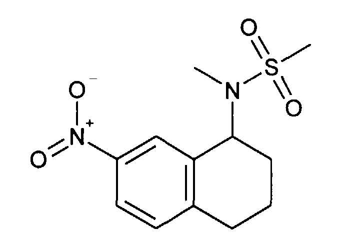

a) 10,0 g (68 mmol) α-Tetralon wurden unter Eiskühlung in 80 ml konz. Schwefelsäure gelöst. Nach portionsweiser Zugabe von 6,4 g (75 mmol) Natriumnitrat wurde noch 1 h bei 0°C gerührt und dann auf 350 ml Eiswasser gegossen. Das ausgefallene Produkt wurde abgesaugt, mit Wasser neutral gewaschen, im Vakuum getrocknet und aus Isopropanol umkristallisiert. Man erhielt 6,5 g 7-Nitro-3,4-dihydro-2H-naphthalin-1-on;
   F.p. 104 - 106°C.
b) Zu einer Lösung von 6,0 g (31 mmol) 7-Nitro-3,4-dihydro-2H-naphthalin-1-on in 150 ml Methanol wurden 24 g (314 mmol) Ammoniumacetat und 13,8 g (220 mmol) Natriumcyanborhydrid gegeben, und die Reaktionsmischung wurde 3 h auf 60°C erhitzt. Nach Ansäuern auf pH <2 mit verd. Salzsäure wurde die Reaktionsmischung im Vakuum eingeengt und der Rückstand mit Wasser und EE verrührt. Ein hierbei auftretender Niederschlag wurde abgesaugt, mit EE gewaschen und dann mit der sauren wäßrigen Phase vereinigt. Nach anschließendem alkalisch stellen wurde mit EE extrahiert und die organische Phase mit Magnesiumsulfat getrocknet und im Vakuum eingeengt. Man erhielt 3,6 g 7-Nitro-1,2,3,4-tetrahydro-1-naphthylamin.
c) Eine Lösung von 2,0 g (10,4 mmol) 7-Nitro-1,2,3,4-tetrahydro-1-naphthylamin in 35 ml THF wurde unter Eiskühlung mit 4,2 g (41,6 mmol) Triethylamin und 1,3 g (11,4 mmol) Methansulfonsäurechlorid versetzt und dann 2 h bei RT gerührt. Nach Zugabe von 20 ml Wasser wurde im Vakuum bis auf 10 ml Rückstand eingeengt, dann mit weiteren 20 ml Wasser versetzt und das ausgefallene Produkt abgesaugt. Nach Trocknen im Vakuum erhielt man 2,5 g N-(7-Nitro-1,2,3,4-tetrahydro-naphthalin-1-yl)-methansulfonamid;
   Fp. 150 - 152°C.
d) Eine Lösung von 1,2 g (4,4 mmol) N-(7-Nitro-1,2,3,4-tetrahydro-naphthalin-1-yl)-methansulfonamid in 16 ml DMF wurde zugetropft zu einer Suspension von 0,15 g (5,1 mmol) 80proz. Natriumhydrid in 10 ml DMF. Nach 1 h Rühren bei RT wurden 0,62 g (4,4 mmol) Iodmethan zugegeben und über Nacht bei RT stehen lassen. Die Reaktionsmischung wurde im Vakuum vollständig eingeengt und der Rückstand dann in EE und Wasser aufgenommen. Nach Waschen der organischen Phase mit verd. Salzsäure und Natriumbicarbonatlösung und Einengen wurde der Rückstand durch Chromatographie an Kieselgel mit Cyclohexan/EE 3:1 gereinigt und man erhielt 0,3 g N-Methyl-N-(7-nitro-1,2,3,4-tetrahydro-naphthalin-1-yl)-methansulfonamid;
   Fp. 138 - 140°C.

### Beispiel 2: N-Hexyl-N-(7-nitro-1,2,3,4-tetrahydro-naphthalin-1-yl)-methansulfonamid

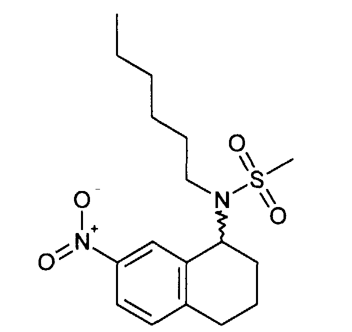

Aus N-(7-Nitro-1,2,3,4-tetrahydro-naphthalin-1-yl)-methansulfonamid (Beispiel 1 c) und 1-Iodhexan wurde analog Beispiel 1d N-Hexyl-N-(7-nitro-1,2,3,4-tetrahydro-naphthalin-1-yl)-methansulfonamid als Öl erhalten.

### Beispiel 3: 5-(Methansulfonyl-methyl-amino)-7,7-dimethyl-5,6,7,8-tetrahydro-naphthalin-1-carbonsäuremethylester

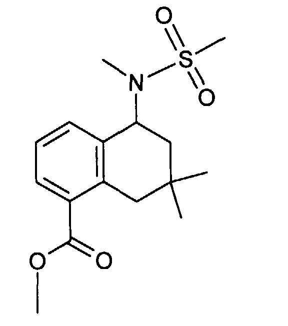

a) Eine Lösung von 3,0 g (13 mmol) 7,7-Dimethyl-5-oxo-5,6,7,8-tetrahydro-1 -naphthalincarbonsäuremethylester (J. Org. Chem. 17, 1976, 2918) in 45 ml Methanol wurde mit 10 g (130 mmol) Ammoniumacetat und 5,7 g (90 mmol) Natriumcyanborhydrid versetzt und 20 h auf 60°C erhitzt. Nach Zugabe von 10 ml Wasser wurde im Vakuum vollständig eingeengt und der Rückstand in EE und wäßrigem Ammoniak aufgenommen. Die organische Phase wurde mit Wasser gewaschen, über Magnesiumsulfat getrocknet und einrotiert, und man erhielt 2,9 g 5-Amino-7,7-dimethyl-5,6,7,8-tetrahydro-1-naphthalincarbonsäuremethylester.
b) Aus 2,8 g 5-Amino-7,7-dimethyl-5,6,7,8-tetrahydro-1-naphthalincarbonsäuremethylester wurden analog Beispiel 1 c 3,1 g 5-Methansulfonylamino-7,7-dimethyl-5,6,7,8-tetrahydro-1-naphthalincarbonsäuremethylester erhalten;
   Fp. 136 - 138°C.
c) Eine Lösung von 0,5 g (1,6 mmol) 5-Methansulfonylamino-7,7-dimethyl-5,6,7,8-tetrahydro-1-naphthalincarbonsäuremethylester in 5 ml DMF wurde zugetropft zu einer Suspension von 0,05 g (1,8 mmol) 80proz. Natriumhydrid in 4 ml DMF. Nach 1 h Rühren bei RT wurden 0,23 g (1,6 mmol) Iodmethan zugegeben und es wurde 4 h bei RT gerührt. Die Reaktionsmischung wurde im Vakuum vollständig eingeengt und der Rückstand dann in EE und Wasser aufgenommen und die organische Phase mit verd. Salzsäure und Natriumbicarbonatlösung gewaschen. Nach Trocknen über Magnesiumsulfat und Einengen im Vakuum wurden 0,5 g 5-(Methansulfonyl-methyl-amino)-7,7-dimethyl-5,6,7,8-tetrahydro-naphthalin-1-carbonsäuremethylester erhalten;
   Fp. 98 - 99°C.

### Beispiel 4: 5-(Hexyl-methansulfonyl-amino)-7,7-dimethyl-5,6,7,8-tetrahydro-naphthalin-1-carbonsäuremethylester

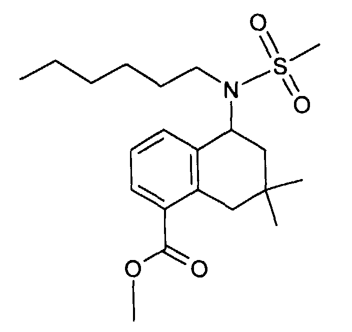

Aus 5-Methansulfonylamino-7,7-dimethyl-5,6,7,8-tetrahydro-1-naphthalincarbonsäuremethylester (Beispiel 3 b) und 1-Iodhexan wurde analog Beispiel 3 c 5-(Hexyl-methansulfonyl-amino)-7,7-dimethyl-5,6,7,8-tetrahydro-naphthalin-1-carbonsäuremethylester als Öl erhalten.

### Beispiel 5: N-(3,3-Dimethyl-1,2,3,4-tetrahydro-naphthalin-1-yl)-N-methyl-methansulfonamid

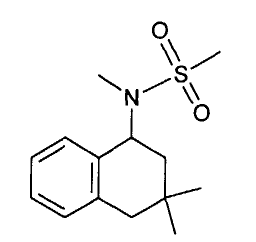

a) Eine Lösung von 2,5 g (11 mmol) 7,7-Dimethyl-5-oxo-5,6,7,8-tetrahydro-1-naphthalincarbonsäuremethylester (J. Org. Chem. 17, 1976, 2918) und 1,8 g (33 mmol) Kaliumhydroxid in 50 ml Methanol und 5 ml Wasser wurde über Nacht bei RT stehen gelassen. Nach Abdestillieren des Methanols im Vakuum wurde der Rückstand in 40 ml Wasser aufgenommen und mit verd. Salzsäure angesäuert. Das ausgefallene Produkt wurde abgesaugt und im Vakuum getrocknet, und man erhielt 2,3 g 7,7-Dimethyl-5-oxo-5,6,7,8-tetrahydro-1-naphthalincarbonsäure;
   Fp. 167 - 168°C.
b) 2,2 g (10 mmol) 7,7-Dimethyl-5-oxo-5,6,7,8-tetrahydro-1-naphthalincarbonsäure wurden mit 2,2 g Kupferpulver in 22 g Chinolin 5 h auf 180°C erhitzt. Nach dem Abkühlen wurde mit EE verdünnt, vom Kupfer abfiltriert und dann mehrfach mit verd. Salzsäure gewaschen. Nach Einrotieren der organischen Phase und Reinigung durch Chromatographie an Kieselgel mit Cycohexan / EE 6 : 1 wurden 0,6 g 3,3-Dimethyl-3,4-dihydro-2H-naphthalin-1-on erhalten.
   ¹H-NMR (200 MHz, CDCl₃): δ [ppm] = 1.1 (s, 6H), 2.5 (s, 2H), 2.85 (s, 2H), 7.25(1H), 7.3 (1H), 7.5 (1H), 8.0 (1H).
c) Aus 3,3-Dimethyl-3,4-dihydro-2H-naphthalin-1-on kann analog Beispiel 3 a - 3 c N-(3,3-Dimethyl-1,2,3,4-tetrahydro-naphthalin-1-yl)-N-methyl-methansulfonamid erhalten werden.

### Beispiel 6: N-(3,3-Dimethyl-7-nitro-1,2,3,4-tetrahydro-naphthalin-1-yl)-N-methyl-methansulfonamid

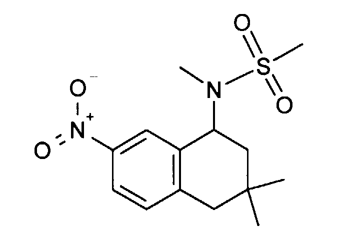

Aus 3,3-Dimethyl-3,4-dihydro-2H-naphthalin-1-on (Beispiel 5b) kann analog Beispiel 1 a - 1 d N-(3,3-Dimethyl-7-nitro-1,2,3,4-tetrahydro-naphthalin-1-yl)-N-methyl-methansulfonamid erhalten werden.

### Beispiel 7: N-Methyl-N-(1,2,3,4-tetrahydronaphthalin-1-yl)-ethansulfonamid

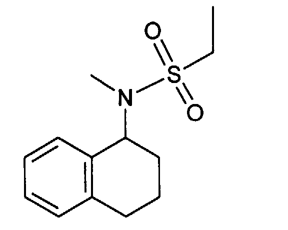

Aus 1-Amino-tetralin wird analog Beispiel 1 c - 1 d (aus Ethansulfonsäurechlorid) das N-Methyl-N-(1,2,3,4-tetrahydro-naphthalin-1-yl)-ethansulfonamid als Öl erhalten.

### Beispiel 8: N-Butyl-N-(1,2,3,4-tetrahydronaphthalin-1-yl)-ethansulfonamid

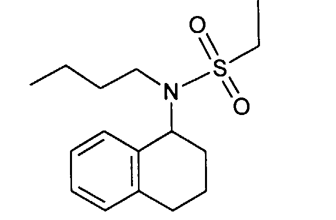

Aus 1-Amino-tetralin wird analog Beispiel 1 c -1 d N-Butyl-N-(1,2,3,4-tetrahydronaphthalin-1-yl)-ethansulfonamid als Öl erhalten.

### Beispiel 9: N-Methyl- N-(1-phenyl-1,2,3,4-tetrahydrochinolin-4-yl)-ethansulfonamid

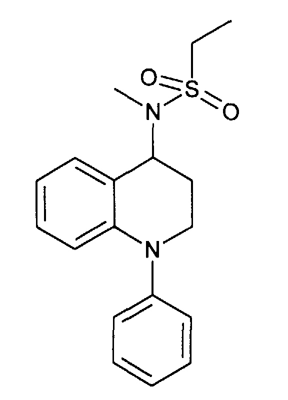

Aus 1-Phenyl-2,3-dihydro-1H-chinolin-4-on wird analog Beispiel 1 a - 1 d das N-Methyl-N-(1-phenyl-1,2,3,4-tetrahydrochinolin-4-yl)-ethansulfonamid als farbloser Feststoff erhalten. Fp. 72°C

### Beispiel 10: N-Butyl-N-(1-phenyl-1,2,3,4-tetrahydrochinolin-4-yl)-ethansulfonamid

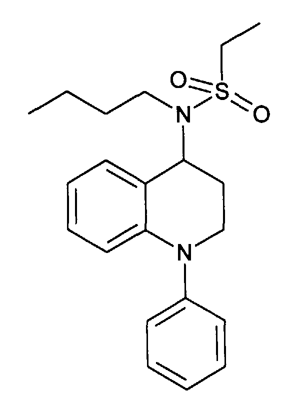

Aus 1-Phenyl-2,3-dihydro-1H-chinolin-4-on wird analog Beispiel 1 a - 1 d das N-Butyl-N-(1-phenyl-1,2,3,4-tetrahydrochinolin-4-yl)-ethansulfonamid als Öl erhalten.

## Patentansprüche

1. Verbindungen der Formel I 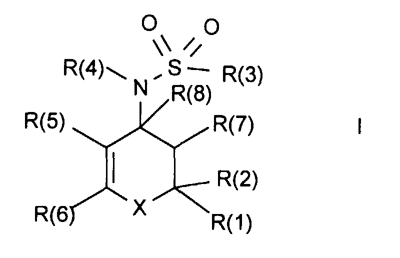 worin bedeuten:
X - [SO_{Null, 1 oder 2}]-, -NR(9)-, -[CR(9)R(23)]- oder -CO-;
R(9) Wasserstoff oder -(CₙH₂ₙ)-R(10);
n Null, 1, 2, 3, 4, 5, 6, 7 oder 8;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
R(11) Wasserstoff, Methyl oder Ethyl;
oder
R(10) Pyridyl, Thienyl, Imidazolyl oder Phenyl, welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
oder
R(9) gemeinsam mit R(1) eine Bindung;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(24) Wasserstoff, Methyl oder Ethyl;
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen,
CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
R(11) Wasserstoff, Methyl oder Ethyl;
R(4) R(14)-CᵣH₂ᵣ;
r 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
R(3) und R(4)
gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
R(5) und R(6)
gemeinsam -CR(15)=CR(16)-CR(17)=CR(18)-, -CR(15)=CR(16)-CR(17)=N-, -CR(15)=CR(16)-N=CR(18)-, -CR(15)=N-CR(17)=N-, -CR(15)=N-N=CR(18)-, -N=CR(16)-CR(17)=N- oder -S-CR(15)=CR(16)-;
R(15), R(16), R(17) und R(18)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1 -Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

2. Verbindungen der Formel I nach Anspruch 1, in welcher bedeuten:
x -[S(O)_{Null, 1 oder 2}]-, -NR(9)- oder -[CR(9)R(23)]- ;
R(9) Wasserstoff oder (CₙH₂ₙ)-R(10);
n Null, 1, 2, 3, 4, 5, 6, 7 oder 8;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -C=C-, -C≡C-, -CO-, -CO-O-, -[SO_{Null, 1 oder 2}]- oder -NR(11)-;
R(11) Wasserstoff, Methyl oder Ethyl;
oder
R(10) Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und
Methylsulfonylamino;
oder
R(9) gemeinsam mit R(1) eine Bindung;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(24) Wasserstoff, Methyl oder Ethyl;
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
R(11) Wasserstoff, Methyl oder Ethyl;
R(4) R(14)-CᵣH₂ᵣ;
r 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
R(3) und R(4)
gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
R(5) und R(6)
gemeinsam -CR(15)=CR(16)-CR(17)=CR(18)- oder
-S-CR(15)=CR(16)-;
R(15), R(16), R(17) und R(18)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und
Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

3. Verbindungen der Formel 1 nach Ansprüchen 1 oder 2, dadurch gekennzeichnet, daß darin bedeuten:
X -NR(9)- oder -[CR(9)R(23)]-;
R(9) Wasserstoff oder -(CₙH₂ₙ)-R(10);
n Null 1, 2, 3, 4, 5, 6, 7 oder 8;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
R(11) Wasserstoff Methyl oder Ethyl;
oder
R(10) Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
oder
R(9) gemeinsam mit R(1) eine Bindung;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(24) Wasserstoff, Methyl oder Ethyl;
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
R(11) Wasserstoff, Methyl oder Ethyl;
R(4) R(14)-CᵣH₂ᵣ;
r 1, 2, 3, 4, 5, 6, 7, 8, 9 oder 10;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
R(3) und R(4)
gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
R(5) und R(6)
gemeinsam -CR(15)=CR(16)-CR(17)=CR(18)-;
R(15), R(16), R(17) und R(18)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und
Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

4. Verbindungen der Formel I nach Ansprüchen 1 bis 3, dadurch gekennzeichnet, daß darin bedeuten:
X -NR(9)- oder -[CR(9)R(23)]-;
R(9) Wasserstoff oder -(CₙH₂ₙ)-R(10);
n Null, 1, 2, 3, 4, 5, 6, 7 oder 8;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, CO-, -CO-O-, -[SO_{Null, 1 oder 2}]- oder -NR(11)-;
R(11) Wasserstoff, Methyl oder Ethyl;
oder
R(10) Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und
Methylsulfonylamino;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
R(11) Wasserstoff, Methyl oder Ethyl;
R(4) R(14]-CᵣH₂ᵣ;
r 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
R(3) und R(4)
gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
R(5) und R(6)
gemeinsam -CR(15)=CR(16)-CR(17)=CR(18)-;
R(15), R(16), R(17) und R(18)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und
Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

5. Verbindungen der Formel I nach Ansprüchen 1 bis 4, dadurch gekennzeichnet, daß darin bedeuten:
X -NR(9)- oder -[CR(9)R(23)]-;
R(9) Wasserstoff oder -(CₙH₂ₙ)-R(10);
n Null, 1, 2, 3 oder 4;
R(10) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅ oder C₃F₇;
wobei eine CH₂-Gruppe der Gruppe CₙH₂ₙ ersetzt sein kann durch -O-, -CH=CH-, -C≡C-, -CO-, -CO-O-, -[SO_{Null, 1 oder 2}]- oder -NR(11)-;
R(11) Wasserstoff, Methyl oder Ethyl;
oder
R(10) Pyridyl, Thienyl, Imidazolyl oder Phenyl,
welche unsubstituiert sind oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
R(23) Wasserstoff, Alkyl mit 1, 2 oder 3 C-Atomen, OH, O-Alkyl mit 1, 2 oder 3 C-Atomen, COOH, COO-Alkyl mit 1, 2 oder 3 C-Atomen oder -CO-R(24);
R(1) und R(2)
unabhängig voneinander Wasserstoff, CF₃, C₂F₅, C₃F₇, Alkyl mit 1 oder 2 C-Atomen;
oder
R(1) und R(2)
gemeinsam eine Alkylenkette mit 2, 3, 4, 5 oder 6 C-Atomen;
R(3) R(12)-CₐH₂ₐ[NR(13)]ₘ-;
R(12) Wasserstoff oder Cycloalkyl mit 3, 4, 5 oder 6 C-Atomen, CF₃,
C₂F₅ oder C₃F₇;
a Null, 1, 2, 3, 4, 5 oder 6;
m Null;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen, wobei eine CH₂-Gruppe der Alkylengruppe durch -O-, -[SO_{Null, 1 oder 2}]-, -CO- oder -NR(11)- ersetzt sein kann;
R(11) Wasserstoff, Methyl oder Ethyl;
R(4) R(14)-CᵣH₂ᵣ;
r 1, 2, 3, 4, 5, 6, 7, 8, 9 oder 10;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino; wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
oder
R(3) und R(4)
gemeinsam eine Alkylenkette mit 3, 4, 5, 6, 7 oder 8 C-Atomen, wobei eine CH₂-Gruppe der Alkylenkette durch -O-, -[SO_{Null, 1 oder 2}]-, -CO- oder -NR(11)- ersetzt sein kann;
R(5) und R(6)
gemeinsam -CR( 15)=CR(16)-CR(17)=CR(18)-;
R(15) und R(18)
Wasserstoff;
R(16) und R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(20), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und
Methylsulfonyl;
R(19) und R(20)
unabhängig voneinander Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen, -COOR(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen.

6. Verwendung einer Verbindung I nach einem oder mehreren der Ansprüche 1 bis 5 zur Herstellung eines Medikaments mit K⁺-Kanal blockierenden Wirkung zur Behandlung oder Prophylaxe von Krankheiten.

7. Verwendung einer Verbindung I nach einem oder mehreren der Ansprüche 1 bis 5 zur Herstellung eines Medikaments zur Behandlung oder Prophylaxe von Herzrhythmusstörungen, die durch Aktionspotential-Verlängerung behoben werden können.

8. Verwendung einer Verbindung I nach einem oder mehreren der Ansprüche 1 bis 5 zur Herstellung eines Medikaments zur Behandlung oder Prophylaxe von atrieller Fibrillation oder atriellem Flattern.

9. Verwendung einer Verbindung I nach einem oder mehreren der Ansprüche 1 bis 5 zur Herstellung eines Medikaments zur Behandlung oder Prophylaxe der stimulierten Magensäuresekretion aus.

10. Verwendung einer Verbindung I nach einem oder mehreren der Ansprüche 1 bis 5 zur Herstellung eines Medikaments zur Behandlung oder Prophylaxe von Ulcera des Magens und des intestinalen Bereiches.

11. Verwendung einer Verbindung I nach einem oder mehreren der Ansprüche 1 bis 5 zur Herstellung eines Medikaments zur Behandlung oder Prophylaxe der Refluxoesophagitis.

12. Verwendung einer Verbindung I nach einem oder mehreren der Ansprüche 1 bis 5 zur Herstellung eines Medikaments zur Behandlung oder Prophylaxe der Diarrhoe.

13. Verwendung einer Verbindung I nach einem oder mehreren der Ansprüche 1 bis 5 zur Herstellung eines Medikaments zur Behandlung oder Prophylaxe aller Typen von Arrhythmien einschließlich ventrikulärer und supraventrikulärer Arrhythmien.

14. Verwendung einer Verbindung I nach einem oder mehreren der Ansprüche 1 bis 5 zur Herstellung eines Medikaments zur Behandlung oder Prophylaxe von Reentry-Arrhythmien und zur Verhinderung des plötzlichen Herztodes infolge von Kammerflimmern.

15. Heilmittel, enthaltend eine wirksame Menge einer Verbindung der Formel I nach Ansprüchen 1 bis 5.
